# EUROPÄISCHE PATENTSCHRIFT

(11) **EP 0 994 843 B1**
(45) Veröffentlichungstag und Bekanntmachung des Hinweises auf die Patenterteilung: **16.01.2002**
(21) Anmeldenummer: 98939542.1
(22) Anmeldetag: 26.06.1998
(51) Int. Cl.: C07C 67/08, C07C 69/54

(54) **VERFAHREN ZUR VERESTERUNG VON (METH)ACRYLSÄURE**
METHOD FOR ESTERIFICATION OF (METH)ACRYLIC ACIDS
PROCEDE POUR L'ESTERIFICATION D'ACIDE (METH) ACRYLIQUE

(30) Priorität: 26.06.1997 DE 19727234
(43) Veröffentlichungstag der Anmeldung: 26.04.2000
(73) Patentinhaber: BASF AKTIENGESELLSCHAFT, 67056 Ludwigshafen (DE)
(72) Erfinder: HENNIG, Gunnar, D-68165 Mannheim (DE); SCHRAUT, Armin, D-64625 Bensheim (DE); MARTIN, Friedrich-Georg, D-69124 Heidelberg (DE); ULBRICH, Michael-Dieter, D-67251 Freinsheim (DE); BAUR, Karl, Gerhard, D-67063 Ludwigshafen (DE); THIESSEN, Fritz, D-67071 Ludwigshafen (DE)
(74) Vertreter: Isenbruck, Günter, Dr.
(86) Internationale Anmeldenummer: EP9803931
(87) Internationale Veröffentlichungsnummer: WO9900354

(56) Entgegenhaltungen:
- EP-A- 0 398 226
- EP-A- 0 594 008
- DE-A- 3 146 191

## Beschreibung

Die vorliegende Erfindung betrifft ein Verfahren zur Herstellung eines Alkyl(meth)acrylats durch kontinuierliche Umsetzung einer direkt aus der Gasphasenoxidation erhaltenen (Meth)acrylsäure-haltigen wäßrigen Lösung mit einem Alkanol.

Der im Rahmen dieser Anmeldung verwendete Begriff "(Meth)acrylsäure" bezeichnet sowohl Acrylsäure als auch Methacrylsäure.

Unter anderem ist Methacrylsäure zugänglich durch Gasphasenoxidation von 1-Buten, Isobuten, Isobutyraldehyd, Isobuttersäure, MTBE und/oder Methacrolein mit Sauerstoff oder Sauerstoff-enthaltenden Gasen in Gegenwart von Katalysatoren, wie z.B. Multimetalloxiden, die die Elemente Molybdän und Vanadium in oxidischer Form enthalten. Vorzugsweise geht man von Methacrolein aus. Die Oxidation wird bei erhöhter Temperatur sowie infolge der hohen Reaktionswärme vorzugsweise unter Verdünnen der Reaktionspartner mit Inertgasen wie N₂, CO₂ und/oder Kohlenwasserstoffen und/oder Wasserdampf durchgeführt. Bei diesem Verfahren wird jedoch nicht reine Methacrylsäure, sondern ein Reaktionsgemisch, das neben Methacrylsäure nicht-umgesetztes Edukt, wie z.B. Methacrolein, Wasserdampf, inertes Verdünnungsgas (z.B. Stickstoff) und Nebenprodukte (z.B. Kohlenoxide), niedere Aldehyde, wie z.B. Formaldehyd, hochsiedende Bestandteile, wie z.B. Citraconsäure, sowie Säuren, wie z.B. Essigsäure, Acrylsäure, Ameisensäure und/oder Propionsäure, insbesondere Essigsäure enthält, erhalten (vgl. z.B. EP-A 0 253 409 und DE-A 19 62 431).

Als Ausgangsverbindungen sind aber auch solche denkbar, aus welchem sich das Edukt, z.B. Methacrolein während der Gasphasenoxidation erst intermediär bildet. Beispielhaft genannt sei der Methylether des tert.-Butanols (MTBE). Es ist auch möglich zur Herstellung von Methacrolein Formaldehyd und Propionaldehyd zu kondensieren und durch anschließende Destillation Methacrolein zu erhalten. Ein derartiges Verfahren wird in der EP-B 0 058 927 beschrieben. Das so gewonnene Methacrolein kann danach in herkömmlicher Weise durch katalytische Gasphasenoxidation zu Methacrylsäure umgesetzt werden. Eine derartige Umsetzung wird u.a. in der EP-B 0 297 445 beschrieben.

Die Gewinnung von Acrylsäure ist u. a. ausgehend von den entsprechenden C₃-Verbindungen, insbesondere Propen und/oder Acrolein, analog möglich.

In der Regel wird das in einem wie oben beschriebenen Verfahren zur Herstellung von (Meth)acrylsäure erhaltene Reaktionsgasgemisch zunächst einer Kondensationsstufe unterworfen, wobei eine (Meth)acrylsäure-haltige wäßrige Lösung erhalten wird.

Die Veresterung von (Meth)acrylsäure mit einem Alkanol, wie z.B. Methanol, zu einem Alkyl(meth)acrylat, wie z.B. Methyl(meth)acrylat und Wasser ist eine Gleichgewichtsreaktion, die mit zunehmenden Wassergehalt immer mehr auf die Eduktseite geschoben wird. Es ist bekannt, daß man solche Gleichgewichtsreaktionen durch Einsatz eines Überschusses einer der Ausgangskomponenten (meistens des Alkanols) oder durch Entfernung der Reaktionsprodukte (Wasser und Alkyl(meth)acrylat) aus dem Veresterungsgemisch auf die Produktseite verschieben kann. Ferner ist bekannt, daß durch Zusatz geeigneter Säuren die Veresterung erheblich beschleunigt werden kann.

So wird in der JP-A 7 301 369 ein Gemisch aus Methacrylsäure mit einem Überschuß an Alkanol (molarer Überschuß 1,2 - 5,0) in Gegenwart eines sauren Katalysators verestert.

Ferner sind zahlreiche Veresterungsverfahren bekannt, in denen eine wäßrige Methacrylsäure-Lösung eingesetzt wird. Dabei wird in der Regel die Methacrylsäure durch Extraktion vom Wasser getrennt und anschließend mit einem Alkanol verestert. Eine destillative Trennung von Methacrylsäure und Wasser ist aufgrund der Azeotropbildung dieser beiden Komponenten nicht möglich.

So beschreibt die DE-A 29 07 602 ein Verfahren zur Herstellung von Alkyl(meth)acrylaten aus verdünnten wäßrigen Methacrylsaure-Lösungen, die durch Extraktion mit einem inerten hochsiedenden Lösungsmittel und anschließender Destillation vom Wasser getrennt werden und dann in Gegenwart eines sauren Katalysators mit einem Alkanol verestert werden.

Diese Verfahren weisen allesamt den Nachteil eines zusätzlichen Trennproblems auf, das durch die Einführung des Extraktionsmittels entsteht und zum Erhalt von (Meth)acrylsäure bzw. des (Meth)acrylsäureesters ohne Reste des Extraktionsmittels eine Reihe von Trennschritten nötig macht.

Ferner beschreibt die US-A 43 31 813 die katalytische Darstellung von Methylmethacrylat aus einer wäßrigen Lösung von lsobuttersäure und Methanol in der Gasphase bei 410 °C und 1 x 10⁵ Pa Druck.

Die direkte Veresterung einer wäßrigen Methacrylsäure-Lösung wird in der DE-A 31 46 191 beschrieben. Gemäß dieser Druckschrift wird ein Veresterungsgemisch aus einer wäßrigen Methacrylsäure-Lösung (Gehalt an Methacrylsäure 5 bis 60 Gew.-%) in Gegenwart von Schwefelsäure oder einer organischen Sulfonsäure kontinuierlich mit einem Alkanol zum entsprechenden Ester umgesetzt und diese anschließend abdestilliert.

EP-A 0 594 008 offenbart ein Verfahren zur Herstellung von Methylmethacrylat, wobei in Gegenwart eines stark sauren Ionenaustauscherharzes Methanol und/oder Methacrylsäure als Flüssigkeit oder teilweise oder ganz gasförmig in einer Flüssigkeit eingesetzt wird.

EP-A 0 398 226 offenbart ein Verfahren zur Herstellung eines Acrylesters durch katalytische Veresterung eines angereicherten Acrylsäurestroms, erhalten durch teilweise Kondensation eines Reaktionsgases aus der katalytischen Oxidation von Propylen und/oder Acrolein.

Die Aufgabe der vorliegenden Erfindung liegt nunmehr darin, ein weiter verbessertes Verfahren zur Herstellung eines Alkyl(meth)acrylats durch kontinuierliche Umsetzung einer direkt aus der Gasphasenoxidation von (Meth)acrolein enthaltenen (Meth)acrylsäure-haitigen wäßrigen Lösung bereitzustellen, das es ermöglicht, wäßrige (Meth)acrylsäure-Lösungen mit einem Wassergehalt von bis zu 90 Gew.-% in Gegenwart von verschiedenen Nebenkomponenten, wie z.B. nicht-umgesetztes Edukt, wie z.B. (Meth)acrolein (bis zu ungefähr 1 Gew.-%). Essigsäure (bis zu ungefähr 10 Gew.-%), Ameisensäure (bis zu ungefähr 1.5 Gew.-%), sowie hochsiedende Bestandteile durch Umsetzung mit einem geeigneten Alkanol in das entsprechende Alkyl(meth)acrylat umzusetzen. Ferner sollte das erfindungsgemaße Verfahren energetisch günstiger betrieben werden können als die bislang bekannten Verfahren.

Diese Aufgaben werden durch das erfindungsgemäße Verfahren erfüllt.

Demgemäß betrifft die vorliegende Erfindung ein Verfahren zur Herstellung eines Alkyl(meth)acrylats durch kontinuierliche Umsetzung einer direkt aus der Gasphasenoxidation erhaltenen (Meth)acrylsäure-haltigen wäßrigen Lösung mit einem gerad-oder verzweigtkettigen Alkanol mit 1 bis 6 C-Atomen oder einem Gemisch aus zwei oder mehr davon in Gegenwart eines Veresterungskatalysators, dadurch gekennzeichnet, daß das Alkanol gasförmig in die (Meth)acrylsäure-haltige wäßrige Lösung eingebracht wird.

Wie bereits oben erwähnt, wird gemäß des erfindungsgemäßen Verfahrens eine direkt aus der Gasphasenoxidation z.B. von (Meth)acrolein erhaltene (Meth)acrylsäure-haltige wäßrige Lösung umgesetzt. Derartige wäßrige Lösungen weisen im allgemeinen einen (Meth)acrylsäure-Gehalt von ungefähr 10 bis ungefähr 90 Gew.-%, vorzugsweise ungefähr 40 bis ungefähr 90 Gew.-% und weiter bevorzugt ungefähr 50 bis ungefähr 90 Gew.-% (Meth)acrylsäure auf. Der Wassergehalt dieser Lösungen beträgt demgemäß im allgemeinen ungefähr 10 bis ungefähr 90 Gew.-%, vorzugsweise ungefähr 10 bis ungefähr 60 Gew.-% und weiter bevorzugt ungefähr 10 bis ungefähr 50 Gew.-%. Als wichtigste Nebenbestandteile sind nicht-umgesetztes Edukt, wie z.B. (Meth)acrolein bis zu einem Gehalt von ungefähr 1 Gew.-%, Essigsäure bis zu einem Gehalt von ungefähr 10 Gew.-% und Ameisensäure bis zu einem Gehalt von 1,5 Gew.-% sowie hochsiedende Bestandteile zu nennen, wobei sich die oben genannten Prozentangaben zu jeweils 100 Gew.-% addieren.

Dabei ist im Rahmen des erfindungsgemäßen Verfahrens insbesondere als überraschend anzusehen, daß es bei Gegenwart von nicht-umgesetztem Edukt, wie z.B. von (Meth)acrolein, also einem Aldehyd mit einer C-C-Doppelbindung als weitere reaktive Gruppe, unter den gewählten Umsetzungsbedingungen nicht zur Bildung von Rückständen in dem Umsetzungsgemisch führt, obwohl bekannt ist, daß z.B. Aldehyde in Gegenwart von Säuren zur Verharzung neigen.

Ferner kann die eingesetzte (Meth)acrylsäure-haltige wäßrige Lösung noch einen geringen Gehalt, d.h. bis zu ungefähr 1 Gew.-%, an schwerflüchtigen Bestandteilen, wie z.B. Polymerisationsinhibitoren aufweisen. Im Laufe der Umsetzung sammeln sich diese schwerflüchtigen Bestandteile im Umsetzungsgemisch an und können beispielsweise durch kontinuierlichen Abzug eines Teils der Sumpfphase entfernt werden. Die Sumpfphase kann, falls dies erwünscht ist, wiederum durch herkömmliche Verfahren, insbesondere Destillationsverfahren, aufgearbeitet werden. Die in der abgetrennten Sumpfphase erhaltenen flüchtigen Bestandteile, wie z.B. Wasser, (Meth)acrylsäure und Alkyl(meth)acrylat, können nach der Abtrennung von den niedrigsiedenden Bestandteilen wiederum der Umsetzung zugeführt werden.

Zur Herstellung der im Rahmen des vorliegenden Verfahrens umgesetzten (Meth)acrylsäure-haltigen wäßrigen Lösung können die aus der Gasphasenoxidation erhaltenen gasförmigen Produkte entweder in Wasser aufgenommen werden, wobei in der Regel Lösungen mit einem relativ hohen Wassergehalt von im allgemeinen mehr als 50 Gew.-% Wasser erhalten werden.

In einer weiteren Ausführungsform können die aus der Gasphasenoxidation erhaltenen gasförmigen Produkte durch Abkühlen kondensiert werden, wobei dann direkt (Meth)acrylsäure-haltige wäßrige Lösungen mit einem relativ geringen Wassergehalt von im allgemeinen bis zu 50 Gew.-% erhalten werden.

Als Katalysator werden Schwefelsäure oder Sulfonsäuren, wie z.B. Benzolsulfonsäuren, insbesondere Dodecylbenzolsulfonsäure, Toluolsulfonsäuren oder Methansulfonsäure verwendet, wobei p-Toluolsulfonsäure bevorzugt ist. Der Anteil des Katalysators im Umsetzungsgemisch wird so bemessen, daß er in einer Konzentration von ungefähr 0,5 bis 2 mol/l, vorzugsweise ungefähr 1 bis 1,5 mol/l Umsetzungsgemisch vorliegt. Obwohl sich der zugegebene Katalysator zum überwiegenden Teil oder sogar vollständig innerhalb der Umsetzung permanent regeneriert, können im Laufe der Umsetzung diskontinuierlich oder kontinuierlich kleinere Mengen an Katalysator nachdosiert werden.

Im Rahmen des erfindungsgemäßen Verfahrens wird die (Meth)acrylsäurehaltige wäßrige Lösung, gegebenenfalls zusammen mit geringen Mengen an Katalysator, kontinuierlich einer Umsetzungsvorrichtung zugeführt, wobei die Menge des im stationären Zustand vorliegenden Umsetzungsgemischs bei 5 bis 200 Gew.-%, vorzugsweise bei 50 bis 150 Gew.-%, bezogen auf die pro Stunde zulaufende (Meth)acrylsäure-haltige wäßrige Lösung, betragen.

Gleichzeitig mit der oben genannten wäßrigen Lösung wird dem Umsetzungsgemisch ein gerad- oder verzweigtkettiges Alkanol mit 1 bis 6 C-Atomen als Gas in wenigstens equimolarer Menge, gegebenenfalls in einem stöchiometrischen Überschuß bis zu einem 4fachen Überschuß, bezogen auf die Menge an zugelaufener (Meth)acrylsäure, kontinuierlich zugesetzt. Das bevorzugte Molverhältnis von (Meth)acrylsäure zu Alkanol liegt bei ungefähr 1 zu ungefähr 1,2 bis ungefähr 2,0. Vorzugsweise werden im Rahmen des erfindungsgemäßen Verfahrens Methanol, Ethanol, Isopropanol, n-Propanol, Isobutanol, tert.-Butanol und n-Butanol, weiter bevorzugt n-Butanol, tert.-Butanol und Methanol als Alkanol eingesetzt. Das eingesetzte Alkanol kann auch eine kleine Wassermenge, vorzugsweise bis zu 20 Gew.-%, besonders bevorzugst 10 Gew.-%, enthalten. Es können selbstverständlich auch Gemische aus zwei oder mehr Alkanolen eingesetzt werden.

Bei der Zuführung des Alkanols werden die Zuführbedingungen, d.h. insbesondere Temperatur und Druck so eingestellt, daß das Alkanol dem Umsetzungsgemisch gasförmig zugeführt wird. Dadurch ist es möglich, den gesamten Energieverbrauch der Veresterung und der anschließenden Verdampfung des Produkts, gegebenenfalls zusammen mit Wasser, verglichen mit einer Zugabe des Alkanols in flüssiger Form, deutlich zu verringern.

Das Umsetzungsgemisch wird gegebenenfalls bei vermindertem Druck und bei Temperaturen zwischen ungefähr 70 und ungefähr 170 °C am Sieden gehalten. Dazu sind im allgemeinen Drücke von ungefähr 6 x 10³ bis 2 x 10⁵ Pa erforderlich. Das Umsetzungsgemisch bildet den Sumpf einer Destillationsanlage, wobei Teile dieses Sumpfes bei Ansammlung von nicht-flüchtigen Rückständen, wie oben definiert, entfernt, gereinigt und gegebenenfalls rückgeführt werden.

Der im Rahmen des erfindungsgemäßen Verfahrens erhaltene Sumpf wird mittels einer geeigneten Heizvorrichtung erhitzt und in einem solchen Maße am Sieden gehalten, daß die Menge des Umsetzungsgemischs ungefähr konstant bleibt. Vorzugsweise ist der Sumpf als Zwangsumlauf-Entspannungsverdampfer oder Umlaufverdampfer ausgebildet, um eine gute Durchmischung des Umsetzungsgemischs ohne mechanische Störung zu gewährleisten.

Das aus dem Sumpf entweichende ein Alkyl(meth)acrylat enthaltende Destillat wird vorzugsweise über eine Destillationskolonne abgenommen. Dieses Destillat enthält ein Gemisch aus dem Alkyl(meth)acrylat-Wasser-Azeotrop und dem eingesetzten Alkanol. Für Methylmethacrylat besitzt das Methylmethacrylat-Wasser-Azeotrop bei Normaldruck die Zusammensetzung 86 Gew.-% Methylmethacrylat und 14 Gew.- % Wasser. Dabei wird vorzugsweise die Destillation so geführt, daß in dem Zulauf für die Destillation in der Regel ebenfalls vorhandene (Meth)acrylsäure vom Destillat getrennt wird. Dies wird insbesondere dann erreicht, wenn ein Teil des gebildeten Alkyl(meth)acrylats in die Destillation rückgeführt wird. Damit liegt das Azeotrop (Meth)aycrylsäure-frei vor, wenn es abgetrennt wird.

Zudem hat es sich erfindungsgemäß als vorteilhaft erwiesen, daß das Azeotrop einphasig vorliegt, wenn es abgetrennt wird. Das Azeotrop liegt dann nicht mehr "einphasig" vor, wenn das Azeoptrop in eine Methyl(meth)acrylatphase und eine Wasserphase aufgetrennt ist. Darüberhinaus hat es sich erfindungsgemäß besonders bewährt, daß das Azeotrop sowohl einphasig als auch (Meth)acrylsäure-frei vorliegt, wenn es abgetrennt wird.

Insbesondere bevorzugt liegt das Azeotrop in der Kolonne einphaisg vor, wodurch eine besonders gute Auftrennung erreicht werden kann.

Falls die Abtrennung mittels einer Destillationskolonne erfolgt, ist es besonders vorteilhaft, daß das Azeotrop im oberen Bereich der Kolonne einphasig oder (Meth)acrylsäure-frei oder vorzugsweise einphasig und (Meth)acrylsäurefrei vorliegt. Dafür haben sich im Kopfbereich der Temperaturen zwischen 50 und 100, bevorzugt 60 und 80 und besonders bevorzugt 65 bis 75°C besonders bewährt. Weiterhin ist es vorteilhaft, daß die Abtrennung unter Normaldruck, vorzugsweise in einem Bereich von ±300 mbar und bevorzugt ± 200 mbar um dem Normaldruck, erfolgt.

Das Azeotrop wird erfindungsgemäß dann als (Meth)acrylsäure-frei bezeichnet, wenn weniger als 10, bevorzugt weniger als 1 und besonders bevorzugt weniger als 0,1 Gew.-% (Meth)acrylsäure im Azeotrop enthalten sind.

Nach dem Kondensieren wird das Destillat in eine obere organische Phase, die das Alkyl(meth)acrylat enthält, und in eine untere Wasserschicht, die das Alkanol enthält, durch eine geeignete Vorrichtung voneinander getrennt, wobei diese Trennung ein- oder mehrstufig durchgeführt werden kann.

Vorzugsweise wird ein Teil, in der Regel der überwiegende Teil, der organischen Phase wieder in eine Kolonne zurückgeführt, ein Teil davon vorzugsweise in den oberen Abschnitt der Kolonne, wodurch die Trennwirkung verbessert wird, um das Alkyl(meth)acrylat in höherer Reinheit zu erhalten.

Die organische Phase kann neben dem Alkyl(meth)acrylat als Hauptprodukt noch hoch- und niedrigsiedende Bestandteile enthalten, die in bekannter Weise durch mehrere Destillationsstufen voneinander getrennt werden können.

Häufig enthält die eingesetzte Methacrylsäure-haltige wäßrige Lösung eine kleine Menge an Isobuttersäure und vor allem Essigsäure. Diese wird gleichzeitig mit der Methacrylsäure verestert und kann aus dem entsprechenden Gemisch der Ester durch Destillation abgetrennt werden, wobei dazu eine Destillationskolonne mit entsprechend großer Bodenzahl benötigt wird.

Die wäßrige Phase des Destillats enthält neben Wasser die Hauptmenge des nicht-umgesetzten Alkanols und entsprechend ihrer Löslichkeit Nebenbestandteile, wie z.B. Aceton oder weitere Aldehyde oder Ketone, sowie Essig- und Ameisensäure. Das Alkanol kann aus der wäßrigen Phase selbstverständlich durch herkömmliche Verfahren, vorzugsweise fraktionierte Destillation, abgetrennt werden und der Umsetzung erneut gasförmig zugeführt werden.

Es ist jedoch erfindungsgemäß bevorzugt, daß der wäßrigen Phase mindestens ein Teil des darin enthaltenden Alkanols entzogen wird. Dieser Entzug erfolgt vorzugsweise durch destillative Abtrennung des Alkanols aus der wäßrigen Phase. Hierzu ist es vorteilhaft, eine Destillationskolonne einzusetzen. Erfindungsgemäß ist bevorzugt mindestens ein Drittel, bevorzugt mindestens die Hälfte und besonders bevorzugt mindestens zwei Drittel des in der wäßrigen Phase enthaltenen Alkanols der wäßrigen Phase zu entziehen. In der Regel weist die wäßrige Phase 1 bis 30, bevorzugt 5 bis 20 und besonders bevorzugt 10 bis 15 Gew.-% des Alkanol, vorzugsweise Methanol, 0,1 bis 10, bevorzugt 1 bis 7 und besonders bevorzugt 2 bis 5 Gew.-% Alkyl(meth)acrylsäureester, vorzugsweise Methyl(meth)acrylat, und bis zu 100 Gew.-% der wäßrigen Phase Wasser auf.

Es hat sich erfindungsgemäß bei der destillativen Abtrennung des Alkanols, und insbesondere bei der destillativen Abtrennung von Methanol, besonders bewährt, daß die wäßrige Phase auf eine Temperatur von 90 bis 130, bevorzugt 95 bis 120 und besonders bevorzugt 100 bis 115°C gehalten wird. Im oberen Bereich der Destillationskolonne hat sich eine Temperatur im Bereich von 60 bis 90, bevorzugt 65 bis 85 und besonders bevorzugt 70 bis 80°C bewährt.

Zudem hat es sich als besonders vorteilhaft erwiesen, daß bei der destillativen Abtrennung des Alkanols ein Druck von 1 bis 3, bevorzugt 1,1 bis 3 und besonders bevorzugt 1,2 bis 1,4 bar herrscht.

Das der wäßrigen Phase entzogene Alkanol kann in die (Meth)acrylsäurehaltige wäßrige Lösung eingebracht werden. Diese Einbringung kann sowohl in Form des flüssigen Alkanols als auch in Form des gasförmigen Alkanols erfolgen, wobei die Einbringung des gasförmigen Alkanols in die (Meth)acrylsäure-haltige wäßrige Lösung bevorzugt ist.

Sowohl der Entzug des Alkanols aus der wäßrigen Phase als auch das Einbringen des Alkanols in die (Meth)acrylsäure-haltige wäßrige Lösung kann kontinuierlich und diskontinuierlich, vorzugsweise kontinuierlich erfolgen.

Mit dem Entzug des Alkanols aus der wäßrigen Phase und der Einbringung des Alkanols in die (Meth)acrylsäure-haltige Lösung ist der Vorteil verbunden, daß auch das nicht abreagiert, in der wäßrigen Phase enthaltene Alkanol der Reaktion in der (Meth)acrylsäure-haltigen wäßrigen Lösung zugeführt werden kann. Somit ist ein sparsamer und Resourcen schonender Einsatz des Alkanols möglich.

Zur Verhinderung einer Polymerisation kann - vorzugsweise am Kopf der Destillationsvorrichtung zur (Meth)acrylsäureabtrennung - ein Polymerisationsinhibitor, wie z.B. Hydrochinon, Phenothiazin oder Hydrochinonmonomethylether zugesetzt werden. Diese gelangen allmählich in den Destillationssumpf und werden bei dessen Aufarbeitung entfernt.

Die vorliegende Erfindung soll nunmehr noch anhand einiger Beispiele erläutert werden.

Die erfindungsgemäßen Beispiele 1 bis 5 wurden in einer Anlage zur kontinuierlichen Veresterung von (Meth)acrylsäure mit Methanol durchgeführt. Diese bestand aus einem Dosierteil, d.h. einem Vorlagebehälter für die (Meth)acrylsäure-haltige wäßrige Lösung, sowie eine Zuführeinrichtung für gasförmiges Methanol, einem Umsetzungsteil mit einem Umsetzungsvolumen von 500 bis 1000 ml, einer Füllkörperkolonne mit einer Länge von 100 cm und einer Nennweite von 5 cm als Destillationseinheit und einer Phasentrennstufe.

Im Umsetzungsteil wurde zu Beginn ein Umsetzungsgemisch aus der direkt aus der Gasphasenoxidation von Methacrolein erhaltenen Methacrylsäure-haltigen wäßrigen Lösung, Methanol und p-Toluolsulfonsäure als Katalysator vorgelegt und auf Betriebsbedingungen gebracht. Anschließend wurde kontinuierlich gasförmige Methanol und die Methacrylsäure-haltige wäßrige Lösung so zudosiert, daß das Sumpfvolumen konstant blieb. Während der Umsetzung gebildetes Methylmethacrylat wurde als Azeotrop mit Wasser über Kopf der Destillationseinheit abgezogen und in einem Phasenabscheider in eine organische und eine wäßrige Phase getrennt. Ein Teil der organischen Phase wurde als Rücklauf auf den Kopf der Destillationskolonne gegeben, um das Abdestillieren von Methacrylsäure zu vermeiden. In den Rücklaufstrom wurde Phenothiazin als Stabilisator zudosiert, um Polymerisation in der Destillationskolonne zu vermeiden.

Die Betriebsbedingungen und Mengenströme, die in den erfindungsgemäßen Bespielen 1 bis 5 verwendet wurden, und die in den Beispielen eingesetzten Methacrylsäure-Konzentrationen sind der beiliegenden Tabelle 1 zu entnehmen.

Die Zusammensetzung der zugeführten Methacrylsäure-haltigen wäßrigen Lösungen zeigt Tabelle 2.

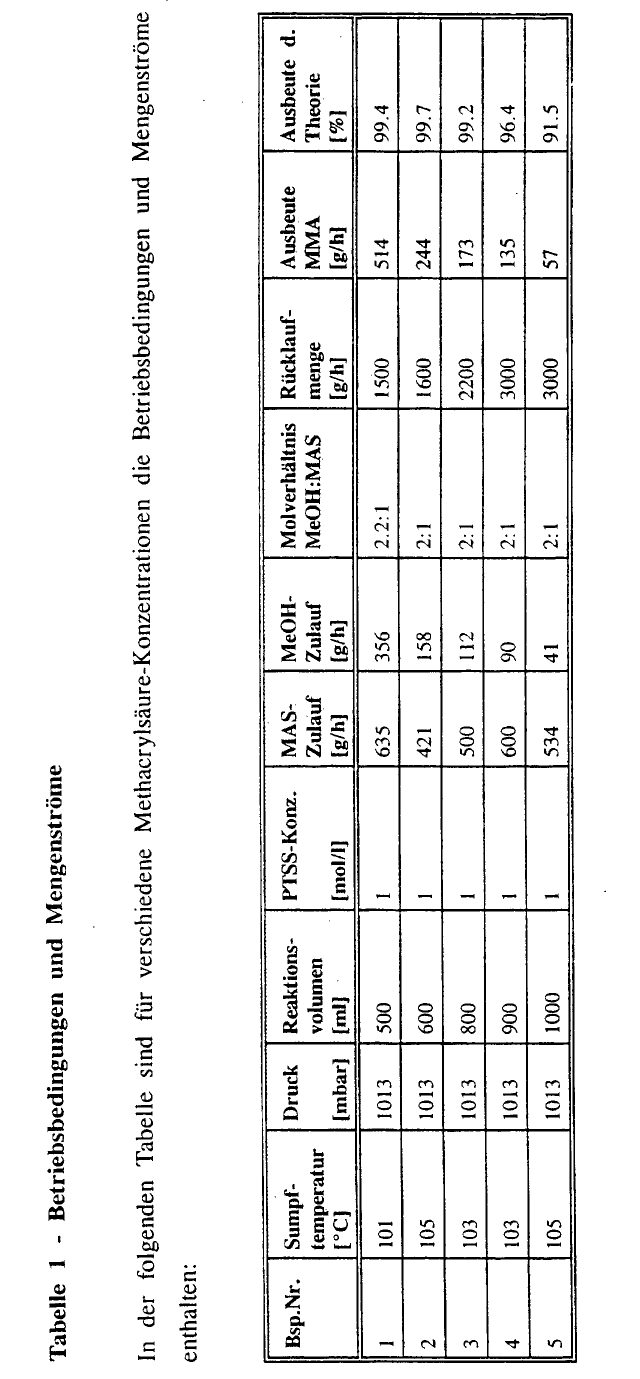

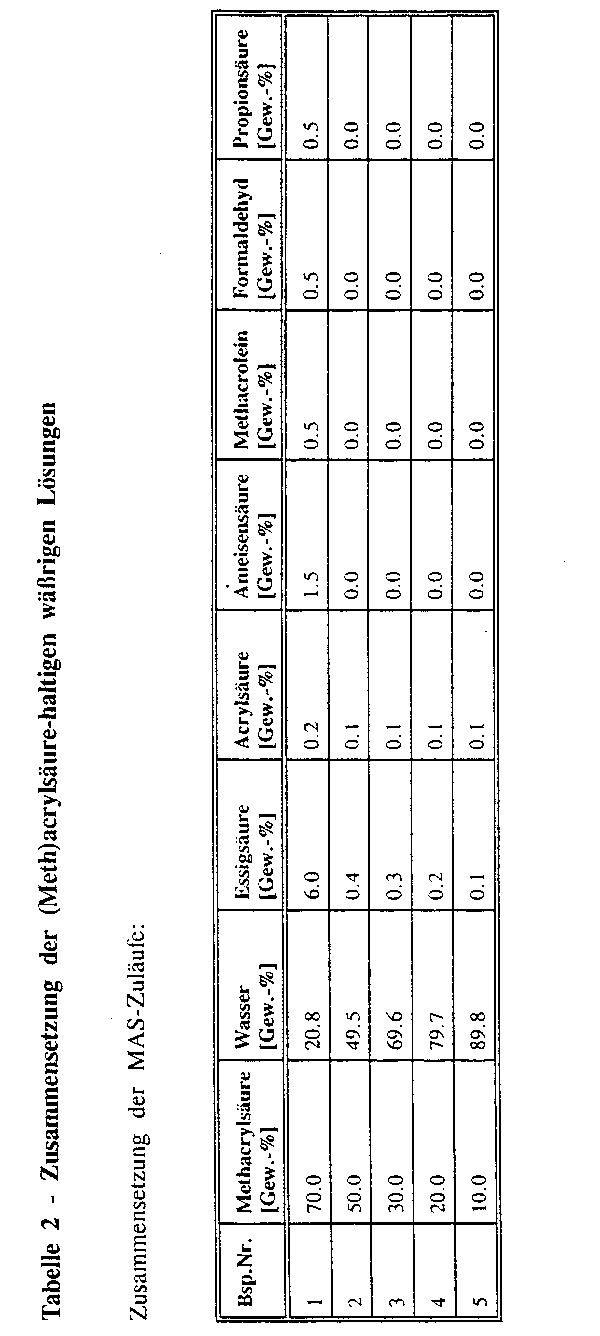

Die Beispiele 6 bis 12 zur kontinuierlichen Veresterung von wäßriger Methacrylsäure wurden in einer Miniplant-Anlage, bestehend aus einem Dosierteil (Vorlagebehälter für Edukte und Stabilisatorlösungen), einem Reaktionsteil (Reaktor mit Umlaufverdampfer), einem Destillationsteil und einer Settlerstufe durchgeführt. In das Reaktionsgefäß wurde zu Beginn 11 der Reaktionslösung vorgelegt und auf Betriebstemperatur erwärmt (95 bis 110°C). In den Reaktor wurde kontinuierlich wäßrige Methacrylsäure-Lösung (flüssig) und wasserhaltiges Methanol (gasförmig) zudosiert. Zusätzlich wurde Stabilisator (Phenothiazin) und Katalysator (p-Toluolsulfonsäure) zugegeben. In der Reaktion gebildetes Methylmethycrylat wurde als Azeotrop mit Wasser, ebenso wie überschüssiges Methanol, über Kopf der Destillationskolonne abgezogen. Das Kondensat des Destillation wurde in einem Phasenschneider in eine wäßrige und eine organische Phase, unter Zugabe von Wasser getrennt. Die organische Phase wurde z.T. als Rücklauf auf den Kopf der Destillationskolonne gegeben. Ein Teil des Sumpfes des Reaktionsteils wurde augfgrund von Nebenreaktionen gelegentlich ausgeschleust.

Die wäßrige Phase wurde in eine Destillationskolonne überführt, in der bei einer Sumpftemperatur von 100 bis 115°C und einer Kopftemperatur von 70 bis 80°C bei 1,3 bar abdestilliert wurde. Der Kopfaustrag enthielt neben Methanol 2 bis 12% Wasser.

Die Betriebsbedingungen und Analyseergebnisse, die sich in den erfindungsgemäßen Beispielen ergaben, sind den beiliegenden Tabellen 3a bis 3c zu entnehmen.

In den folgenden Tabellen sind für unterschiedliche Sumpftemperaturen, MAS:MeOH-Verhältnisse und Rücklaufmengen die Betriebsbedingungen und Analyseergebnisse enthalten:

**Tabelle 3a**

| **Zulauf Temperaturmeßstellen** | | | | | | | | |
|---|---|---|---|---|---|---|---|---|
| Bsp.Nr. | MAS [ml/h] | MeOH [ml/h] | MMA [ml/h] | H₂O [ml/h] | Rücklauf [ml/h] | Sumpf [°C] | Kol.kopf [°C] | nach Kühler [°C] |
| 6 | 600 | 450 | 140 | 750 | 790 | 104 | 71 | 24 |
| 7 | 603 | 429 | 148 | 760 | 800 | 105 | 71 | 24 |
| 8 | 600 | 439 | 155 | 775 | 818 | 105 | 71 | 24 |
| 9 | 600 | 420 | 141 | 750 | 604 | 103 | 71 | 24 |
| 10 | 605 | 426 | 143 | 761 | 630 | 103 | 71 | 25 |
| 11 | 623 | 428 | 140 | 760 | 600 | 107 | 71 | 24 |
| 12 | 588 | 481 | 146 | 748 | 1522 | 115 | 71 | 24 |

**Tabelle 3b**

| **Analyse des organischen Kopfaustrags** | | | | | |
|---|---|---|---|---|---|
| Bsp.Nr. | MeOH in Gew.-% | MeAc in Gew.-% | MMA in Gew.-% | MAS in Gew.-% | Wasser in Gew.-% |
| 6 | 2,12 | 0,39 | 96,04 | 0,00 | 1,45 |
| 7 | 0,86 | 0,28 | 97,43 | 0,00 | 1,44 |
| 8 | 1,44 | 0,25 | 96,87 | 0,00 | 1,44 |
| 9 | 0,40 | 0,25 | 97,92 | 0,00 | 1,43 |
| 10 | 1,32 | 0,24 | 97,07 | 0,00 | 1,38 |
| 11 | 1,60 | 0,23 | 96,71 | 0,00 | 1,45 |
| 12 | 2,02 | 0,46 | 95,94 | 0,00 | 1,58 |

**Tabelle 3c**

| **Analyse des wässrigen Kopfaustrags** | | | | | |
|---|---|---|---|---|---|
| Bsp.Nr. | MeOH in Gew.-% | MeAc in Gew.-% | MMA in Gew.-% | MAS in Gew.-% | Wasser in Gew.-% |
| 6 | 11,12 | 0,06 | 1,60 | 0,01 | 87,21 |
| 7 | 10,43 | 0,04 | 1,62 | 0,00 | 87,91 |
| 8 | 9,31 | 0,04 | 1,66 | 0,01 | 88,99 |
| 9 | 9,54 | 0,04 | 1,63 | 0,01 | 88,79 |
| 10 | 10,02 | 0,03 | 1,63 | Spuren | 88,32 |
| 11 | 10,82 | 0,04 | 1,73 | 0,00 | 87,41 |
| 12 | 11,15 | 0,08 | 1,74 | 0,01 | 87,03 |

Dabei bedeuten Tabellen 3a bis 3c:
- MAS-Zulauf: Lösung von Methacrylsäure in 15 Gew.-% Wasser
- MeOH-Zulauf: gasförmige Eindosierung von Methanol mit 10 Gew.-% Wasser
- H₂O: Zulauf von Wasser in die Settlerstufe zur besseren Phasentrennung des Kondensats

## Patentansprüche

1. Verfahren zur Herstellung eines Alkyl(meth)acrylats durch kontinuierliche Umsetzung einer direkt aus der Gasphasenoxidation erhaltenen (Meth)acrylsäure-haltigen wäßrigen Lösung mit einem gerad- oder verzweigtkettigen Alkanol mit 1 bis 6 C-Atomen oder einem Gemisch aus zwei oder mehr davon in Gegenwart eines Veresterungskatalysators, **dadurch gekennzeichnet, daß** das Alkanol gasförmig in die (Meth)acrylsäure-haltige wäßrige Lösung eingebracht wird.

2. Verfahren nach Anspruch 1, wobei das Alkanol ausgewählt wird aus der Gruppe bestehend aus Methanol, Butanol, tert.-Butanol oder einem Gemisch aus zwei oder mehr davon.

3. Verfahren nach Anspruch 1 oder 2, wobei der Veresterungskatalysator p-Toluolsulfonsäure ist.

4. Verfahren nach einem der Ansprüche 1 bis 3, wobei das Alkyl(meth)acrylat in einer Abtrennstufe als Destillat, das das Azeotrop des Alkyl(meth)acrylats mit Wasser enthält, abgetrennt wird.

5. Verfahren nach Anspruch 4, wobei das Azeotrop einphasig oder (Meth)acrylsäure-frei oder einphasig und (Meth)acrylsäure-frei vorliegt.

6. Verfahren nach Anspruch 4 oder 5, wobei das Destillat in eine organische und eine wäßrige Phase aufgetrennt wird.

7. Verfahren nach Anspruch 6, wobei mindestens ein Teil der organischen Phase in die Abtrennstufe rückgeführt wird.

8. Verfahren nach Anspruch 6, wobei der wäßrigen Phase mindestens ein Teil des darin enthaltenen Alkanols entzogen wird.

9. Verfahren nach Anspruch 8, wobei das Alkanol in die (Meth)acrylsäurehaltige wäßrige Lösung eingebracht wird.

10. Verfahren nach einem der Ansprüche 1 bis 9, wobei die Umsetzung in einem Umlaufverdampfer durchgeführt wird.

## Claims

1. A process for preparing an alkyl (meth)acrylate by continuous reaction of a (meth)acrylic acid-containing aqueous solution obtained directly from the gas-phase oxidation with a linear or branched alkanol having from 1 to 6 carbon atoms or a mixture of two or more thereof in the presence of an esterification catalyst, wherein the alkanol is introduced in gaseous form into the (meth)acrylic acid-containing aqueous solution.

2. A process as claimed in claim 1, wherein the alkanol is selected from the group consisting of methanol, butanol, tert-butanol and mixtures of two or more thereof.

3. A process as claimed in claim 1 or 2, wherein the esterification catalyst is p-toluenesulfonic acid.

4. A process as claimed in any of claims 1 to 3, wherein the alkyl (meth)acrylate is, in a separation stage, separated off as distillate comprising the azeotrope of the alkyl (meth)acrylate with water.

5. A process as claimed in claim 4, wherein the azeotrope is a single phase or is free of (meth)acrylic acid or is both a single phase and free of (meth)acrylic acid.

6. A process as claimed in claim 4 or 5, wherein the distillate is separated into an organic and an aqueous phase.

7. A process as claimed in claim 6, wherein at least a part of the organic phase is returned to the separation stage.

8. A process as claimed in claim 6, wherein at least part of the alkanol present in the aqueous phase is separated off therefrom.

9. A process as claimed in claim 8, wherein the alkanol is introduced into the (meth)acrylic acid-containing aqueous solution.

10. A process as claimed in any of claims 1 to 9, wherein the reaction is carried out in a convection vaporizer.

## Revendications

1. Procédé de préparation d'un (méth)acrylate d'alkyle par réaction en continu d'une solution aqueuse contenant de l'acide (méth)acrylique que l'on obtient directement par l'oxydation en phase gazeuse avec un alcanol à chaîne linéaire ou ramifiée comportant de 1 à 6 atomes de carbone, ou un mélange de deux ou plus de deux d'entre eux, en présence d'un catalyseur d'estérification, **caractérisé en ce que** l'alcanol est introduit sous forme de gaz dans la solution aqueuse contenant de l'acide (méth)acrylique.

2. Procédé selon la revendication 1, dans lequel l'alcanol est choisi dans le groupe formé par le méthanol, le butanol, le tert.-butanol ou un mélange de deux ou plus de deux d'entre eux.

3. Procédé selon la revendication 1 ou 2, où le catalyseur d'estérification est de l'acide p-toluène-sulfonique.

4. Procédé selon l'une des revendications 1 à 3, où le (méth)acrylate d'alkyle est séparé, dans une étape de séparation, en tant que distillat contenant l'azéotrope du (méth)acrylate d'alkyle et de l'eau.

5. Procédé selon la revendication 4, où l'azéotrope est monophase ou exempt d'acide (méth)acrylique, ou bien monophase et exempt d'acide (méth)acrylique.

6. Procédé selon la revendication 4 ou 5, où le distillat est séparé en une phase organique et une phase aqueuse.

7. Procédé selon la revendication 6, où au moins une partie de la phase organique est recyclée dans l'étape de séparation.

8. Procédé selon la revendication 6, où l'on extrait de la phase aqueuse au moins une partie de l'alcanol qu'elle contient.

9. Procédé selon la revendication 8, où l'alcanol est introduit dans la solution aqueuse d'acide (méth)acrylique.

10. Procédé selon l'une des revendications 1 à 9, où la réaction est entreprise dans un évaporateur à circulation.
